# EUROPÄISCHE PATENTSCHRIFT

(11) **EP 1 199 309 B1**
(45) Veröffentlichungstag und Bekanntmachung des Hinweises auf die Patenterteilung: **07.03.2007**
(21) Anmeldenummer: 01124076.9
(22) Anmeldetag: 10.10.2001
(51) Int. Cl.: C07D 498/22, C09B 19/02, C09B 67/00

(54) **Verfahren zur Herstellung neuer Kristallmodifikationen eines methylsubstitutierten Benzimidazolon-Dioxazin-Pigmentes**
Process for producing new crystalline modifications of a methyl-substituted benzimidazolonedioxazine pigment
Procédé de préparation de nouvelles modifications cristallines d'un pigment benzimidazolonedioxazine méthyl-substitué

(30) Priorität: 21.10.2000 DE 10052221
(43) Veröffentlichungstag der Anmeldung: 24.04.2002
(73) Patentinhaber: Clariant Produkte (Deutschland) GmbH, 65929 Frankfurt am Main (DE)
(72) Erfinder: Schmidt, Martin U., Dr., 65931 Frankfurt am Main (DE); Kempter, Peter, Dr., 65812 Bad Soden (DE); Born, Roland, Dr., 68128 Village-Neuf (FR)

(56) Entgegenhaltungen:
- EP-A- 0 911 337
- DE-A- 1 619 389
- DE-A- 3 211 607
- DE-A- 4 442 291

## Beschreibung

Die Erfindung betrifft eine neue Kristallmodifikation von methylsubstituiertem Benzimidazolon-Dioxazin-Pigment der Formel (1)

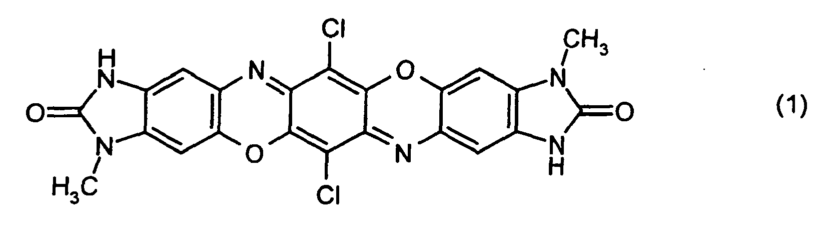

Die Verbindung der Formel (1) wurde erstmals in der DE-A-44 42 291 beschrieben. Eine alternative Synthese analoger Verbindungen wird in EP-A-0 911 337 beschrieben.

Die meisten organischen Pigmente existieren in mehreren verschiedenen Kristallmodifikationen, auch "polymorphe Formen" genannt. Kristallmodifikationen haben dieselbe chemische Zusammensetzung, aber eine unterschiedliche Anordnung der Bausteine (Moleküle) im Kristall. Die Kristallstruktur bestimmt die chemischen und physikalischen Eigenschaften, daher unterscheiden sich die einzelnen Kristallmodifikationen oftmals in der Rheologie, der Farbe und anderen coloristischen Eigenschaften. Die unterschiedlichen Kristallmodifikationen können durch Röntgenpulverdiffraktometrie identifiziert werden.

Vom Dioxazin-Pigment der Formel (1) sind bisher zwei Kristallmodifikationen bekannt, die im Folgenden als Phase I und Phase III bezeichnet werden und sich durch folgende charakteristische Linien im Röntgenpulverdiagramm auszeichnen (Cu-K_{α}-Strahlung, doppelte Beugungswinkel 20 in Grad, Netzebenenabstände d in Å⁻¹):

| Phase I: | | |
|---|---|---|
| 2Θ | d | rel. Intensität |
| 5,00 | 17,68 | 28 |
| 6,08 | 14,53 | 14 (breit) |
| 10,58 | 8,35 | 24 |
| 10,98 | 8,05 | 24 |
| 20,38 | 4,35 | 11 (breit) |
| 22,20 | 4,00 | 10 (breit) |
| 25,18 | 3,53 | 11 (breit) |
| 27,16 | 3,28 | 100 |

| Phase III: | | |
|---|---|---|
| 2Θ | d | rel. Intensität |
| 5,22 | 16,92 | 23 breit |
| 6,18 | 14, 30 | 24 breit |
| 10,92 | 8,09 | 17 breit |
| 11,81 | 7,49 | 30 breit |
| 12,34 | 7,17 | 35 breit |
| 14,32 | 6,18 | 13 breit |
| 17,07 | 5,19 | 10 breit |
| 20,31 | 4,37 | 12 breit |
| 23,16 | 3,84 | 14 breit |
| 25,64 | 3,47 | 18 breit |
| 26,99 | 3,30 | 100 |

Die mit "breit" gekennzeichneten Linienlagen sind mit einer Ungenauigkeit von ± 0.4° behaftet, alle übrigen Linienlagen mit einer Ungenauigkeit von ± 0.2°.

Die Phasen I und III entstehen bei der Synthese des Dioxazinpigmentes der Formel (1) gemäß DE-A-44 42 291 oder EP-A-0 911 337.

Es wurde nun überraschenderweise gefunden, dass sich neue Kristallmodifikationen bilden, wenn man das Pigment der Formel (1) mit bestimmten organischen Lösemitteln behandelt. Dies ist um so mehr überraschend, als beim analogen Pigment der Formel (2) durch Behandeln mit organischen Lösemitteln keine neuen Kristallphasen entstehen.

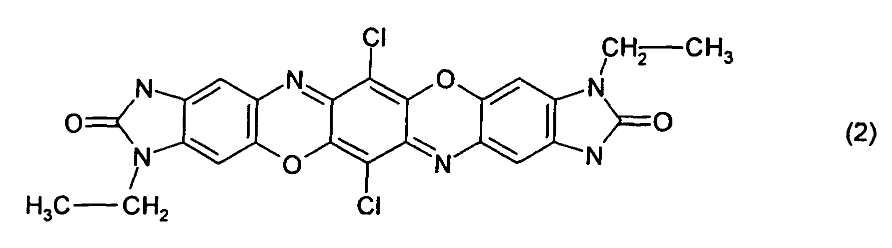

Die neue Kristallmodifikation des Dioxazinpigmentes der Formel (1) wird als Phase II bezeichnet.
Sie zeichnet sich durch folgende charakteristische Linien aus (Cu-K_{α}-Strahlung, 2Θ-Werte in Grad, d-Werte in Å⁻¹):

| Phase II: | | |
|---|---|---|
| 2Θ | d | rel. Intensität |
| 6,64 | 13,29 | 21 |
| 11,05 | 8,00 | 8 |
| 12,49 | 7,08 | 46 |
| 13,41 | 6,60 | 6 |
| 14,60 | 6,06 | 10 |
| 17,08 | 5,19 | 7 |
| 19,87 | 4,46 | 7 |
| 20,93 | 4,24 | 4 |
| 22,24 | 3,99 | 4 |
| 23,35 | 3,81 | 8 |
| 25,36 | 3,51 | 11 |
| 26, 90 | 3, 31 | 100 |
| 29,06 | 3,07 | 5 |
| 30,48 | 2,93 | 8 |
| 32,11 1 | 2,79 | 8 |
| 33,17 | 2,70 | 4 |

Alle Linienlagen sind mit einer Ungenauigkeit von ± 0,2° behaftet.

Die genannte Phase ist äußerst schwerlöslich und zeichnet sich durch gute Echtheiten und violette Färbungen aus.

Im festen Zustand kann die Verbindung der Formel (1) auch in einer anderen tautomeren und/oder isomeren Form vorliegen.

Gegenstand der Erfindung ist ein Verfahren zur Phasenumwandlung einer Verbindung der Formel (1) 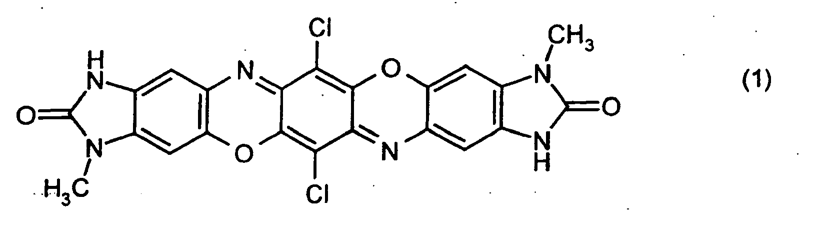 oder einem Tautomeren davon, dadurch gekennzeichnet, dass man auf die Verbindung der Formel (1) N-Methylpyrrolidon, bei einer Temperatur zwischen 0 und 250°C, vorzugsweise zwischen 20 und 210 °C, einwirken lässt.

Zweckmäßigerweise wird das erfindungsgemäße Verfahren so durchgeführt, dass die Verbindung der Formel (1) in dem organischen Lösemittel suspendiert oder teilweise oder vollständig gelöst wird, und das Gemisch bei der genannten Temperatur für 10 Minuten bis 48 Stunden, vorzugsweise 30 Minuten bis 24 Stunden, gehalten wird.

Ausgangsprodukt zur Herstellung der erfindungsgemäßen Phasen ist vorzugsweise die Verbindung der Formel (1) in der Phase I, jedoch ist es auch möglich, jeweils eine oder mehrere der Kristallphasen mit den beschriebenen Maßnahmen in eine der anderen genannten Kristallphasen umzuwandeln.

Die Phase II erhält man beispielsweise, wenn man das Pigment der Formel (1) mit N-Methylpyrrolidon zum Sieden erhitzt.

In Abhängigkeit von der Reinheit der Edukte, den Konzentrationen, den angewandten Temperaturen und Temperaturverläufen, einer eventuellen Nachbehandlung, dem Druck, der Anwesenheit von Verunreinigungen oder Additiven, und der Gegenwart von Impfkristallen kann die reine Phase II, oder eine Mischung mehrerer Phasen, entstehen. Gegenstand der vorliegenden Erfindung ist daher auch eine Mischung von Pigmenten der Formel (1), die mindestens 10 %, vorzugsweise mindestens 25 %, insbesondere mindestens 50 %, besonders bevorzugt mindestens 75 %, ganz besonders bevorzugt mindestens 90 % der Phase II, enthält.

Je nach gewünschtem Anwendungsbereich kann es sinnvoll sein, das erhaltene Pigment einer mechanischen Feinverteilung zu unterwerfen. Die Feinverteilung kann durch Nass- oder Trockenmahlung oder Kneten erfolgen. An die Mahlung bzw. Knetung kann sich eine Behandlung mit einem Lösemittel, mit Wasser, oder einem Lösemittel/Wasser-Gemisch anschließen.

Zur Erleichterung des Modifikationswechsels, zur Stabilisierung der erfindungsgemäßen Modifikation, zur Verbesserung der coloristischen Eigenschaften und zur Erzielung bestimmter coloristischer Effekte können an beliebigen Stellen des Verfahrens Pigmentdispergatoren, oberflächenaktive Mittel, Entschäumer, Extender oder andere Zuschlagstoffe zugesetzt werden. Es können auch Mischungen dieser Zusatzstoffe verwendet werden. Die Zugabe der Zusatzstoffe kann auf einmal oder in mehreren Portionen erfolgen. Die Zusatzstoffe können an jedem Punkt der Synthese oder der verschiedenen Nachbehandlungen, oder nach den Nachbehandlungen zugegeben werden. Der am besten geeignete Zeitpunkt muss zuvor durch orientierende Versuche ermittelt werden.

Das Dioxazinpigment der Formel (1) in der Phase II, oder eine Mischung, die diese Phasen enthält, eignet sich zum Pigmentieren von Lacken, Kunststoffen und Kosmetika, zur Herstellung von Druckfarben, z.B. für Papier- und Textildruck, und Pigmentpräparationen, für die Papiermassefärbung und zur Einfärbung von Saatgut.

Die genannten Phasen und Phasenmischungen des Dioxazinpigmentes der Formel (1) sind geeignet als Farbmittel in elektrophotographischen Tonern und Entwicklern, wie z.B. Ein- oder Zweikomponentenpulvertonern (auch Ein- oder Zweikomponenten-Entwickler genannt), Magnettoner, Flüssigtoner, Latextoner, Polymerisationstoner sowie Spezialtoner.

Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Des weiteren ist die genannte Phase und Phasenmischungen des Dioxazinpigmentes der Formel (1) geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen.

Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem ist die genannte Phase und Phasenmischungen als Farbmittel in Tinten, vorzugsweise Ink-Jet Tinten, wie z.B. auf wässriger oder nichtwässriger Basis, Mikroemulsionstinten sowie in solchen Tinten, die nach dem Hot-melt-Verfahren arbeiten, geeignet.

Ink-Jet-Tinten enthalten im allgemeinen insgesamt 0,5 bis 15 Gew.-%, vorzugsweise 1,5 bis 8 Gew.-%, (trocken gerechnet) der erfindungsgemäßen Verbindung.
Mikroemulsionstinten basieren auf organischen Lösemitteln, Wasser und ggf. einer zusätzlichen hydrotropen Substanz (Grenzflächenvermittler). Mikroemulsionstinten enthalten 0,5 bis 15 Gew.-%, vorzugsweise 1,5 bis 8 Gew.-%, der erfindungsgemäßen Verbindung, 5 bis 99 Gew.-% Wasser und 0,5 bis 94,5 Gew.-% organisches Lösungsmittel und/oder hydrotrope Verbindung.

"Solvent based" Ink-Jet-Tinten enthalten vorzugsweise 0,5 bis 15 Gew.-% erfindungsgemäßer Verbindung, 85 bis 99,5 Gew.-% organisches Lösungsmittel und/oder hydrotrope Verbindungen.

Hot-Melt-Tinten basieren meist auf Wachsen, Fettsäuren, Fettalkoholen oder Sulfonamiden, die bei Raumtemperatur fest sind und bei Erwärmen flüssig werden, wobei der bevorzugte Schmelzbereich zwischen ca. 60°C und ca. 140°C liegt. Hot-Melt Ink-Jet-Tinten bestehen z.B. im wesentlichen aus 20 bis 90 Gew.-% Wachs und 1 bis 10 Gew.-% der erfindungsgemäßen Verbindung. Weiterhin können 0 bis 20 Gew.-% eines zusätzlichen Polymers (als "Farbstofflöser"), 0 bis 5 Gew.-% Dispergierhilfsmittel, 0 bis 20 Gew.-% Viskositätsveränderer, 0 bis 20 Gew.-% Plastifizierer, 0 bis 10 Gew.-% Klebrigkeitszusatz, 0 bis 10 Gew.-% Transparenzstabilisator (verhindert z.B. Kristallisation der Wachse) sowie 0 bis 2 Gew.-% Antioxidans enthalten sein. Typische Zusatzstoffe und Hilfsmittel sind z.B. in US-PS 5,560,760 beschrieben.

Außerdem ist die erfindungsgemäße Verbindung auch geeignet als Farbmittel für Farbfilter, sowohl für die additive wie für die subtraktive Farberzeugung und für "elektronische Tinten".

In den folgenden Beispielen sind Teile und Prozentangaben auf das Gewicht bezogen. Die Bestimmung der Kristallmodifikation der erhaltenen Produkte erfolgt durch Röntgenpulverdiffraktometrie.

### Beispiele

### Vergleichsbeispiel:

Die Synthese des Pigments der Formel (1) erfolgt in Analogie zu der in EP-A-0 911 337, Beispiel 3, beschriebenen Synthesemethode. Man erhält das Pigment der Formel (1) in der Phase I.

### Beispiel 1: Herstellung der Phase II durch Erhitzen in NMP

40 Teile Pigment der Formel (1), welches in der Phase I vorliegt, werden in 400 Teilen N-Methyl-2-pyrrolidon 18 Stunden bei 203°C erhitzt, auf Raumtemperatur abgekühlt, filtriert und anschließend mit Wasser gewaschen. Man erhält 38,1 Teile Pigment der Formel (1) in der Phase II.

### Anwendungsbeispiele:

Zur Beurteilung der Eigenschaften der nach der Erfindung hergestellten PigmentPhase auf dem Lacksektor wurde aus der Vielzahl der bekannten Lacke ein aromatenhaltiger Alkydmelaminharzlack (AM) auf Basis eines mittelöligen Alkydharzes und eines butanolveretherten Melaminharzes ausgewählt.

### Anwendungsbeispiel 1

Eine Applikation des Pigmentes der Phase II aus Beispiel 1 in AM-Lack zeigt eine farbstarke, reine, violette Lackierung, die reiner und farbstärker ist als die von Phase I und wesentlich blauer als die von Phase III.

## Patentansprüche

1. Verfahren zur Phasenumwandlung einer Verbindung der Formel (1) in ein Dioxazin-Pigment gemäß Anspruch 4 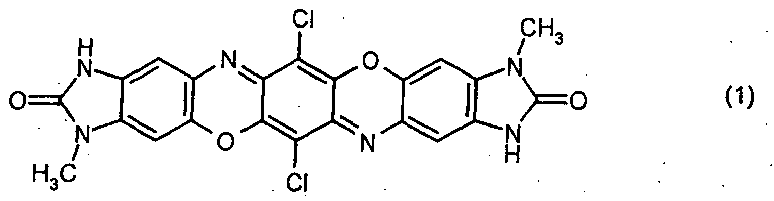 oder einem Tautomeren davon, **dadurch gekennzeichnet, dass** man auf die Verbindung der Formel (1) N-Methylpyrrolidon, bei einer Temperatur zwischen 0 und 250°C einwirken lässt.

2. Verfahren nach Anspruch 1, **dadurch gekennzeichnet, dass** die Phasenumwandlung bei einer Temperatur zwischen 20 und 210°C erfolgt.

3. Verfahren nach Anspruch 1 oder 2, **dadurch gekennzeichnet, dass** die Verbindung der Formel (1) in N-Methylpyrrolidon zum Sieden erhitzt wird.

4. Dioxazin-Pigment der Formel (1) 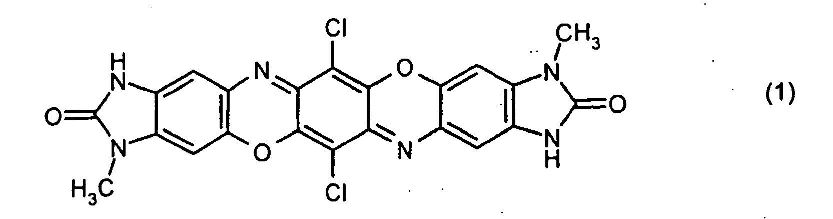 oder ein Tautomeres hiervon, **gekennzeichnet durch** folgende charakteristische Reflexe im Röntgenpulverdiagramm, gemessen mit Cu-K_{α}-Strahlung (2Θ-Werte in Grad, d-Werte in Å⁻¹):
| **Phase II:** | | |
|---|---|---|
| 2Θ | d | rel. Intensität |
| 6,64 | 13,29 | 21 |
| 11,05 | 8,00 | 8 |
| 12,49 | 7,08 | 46 |
| 13,41 | 6,60 | 6 |
| 14,60 | 6,06 | 10 |
| 17,08 | 5,19 | 7 |
| 19,87 | 4,46 | 7 |
| 20,93 | 4,24 | 4 |
| 22,24 | 3,99 | 4 |
| 23,35 | 3,81 | 8 |
| 25,36 | 3,51 | 11 |
| 26,90 | 3,31 | 100 |
| 29,06 | 3,07 | 5 |
| 30,48 | 2,93 | 8 |
| 32,11 | 2,79 | 8 |
| 33,17 | 2,70 | 4 |

5. Mischung eines Pigmentes der Formel (1) nach Anspruch 4, die mindestens 10 %, der Phase II, enthält.

6. Mischung nach Anspruch 5, die mindestens 25% der Phase II enthält.

7. Mischung nach Anspruch 5, die mindestens 50% der Phase II enthält.

8. Mischung nach Anspruch 5, die mindestens 75% der Phase II enthält.

9. Mischung nach Anspruch 5, die mindestens 90% der Phase II enthält.

10. Verwendung eines Pigmentes der Formel (1) nach mindestens einem der Ansprüche 4 bis 9 zum Pigmentieren von Lacken, Kunststoffen, Druckfarben, wässrigen oder lösemittelhaltigen Pigmentpräparationen, Kosmetika, elektrophotographischen Tonern und Entwicklern, Pulverlacken, Tinten, vorzugsweise Ink-Jet-Tinten, Farbfiltern, zum Einfärben von Saatgut und zur Papiermassefärbung.

## Claims

1. A process for phase conversion of a.compound of the formula (1) to a dioxazine pigment as claimed in claim 4 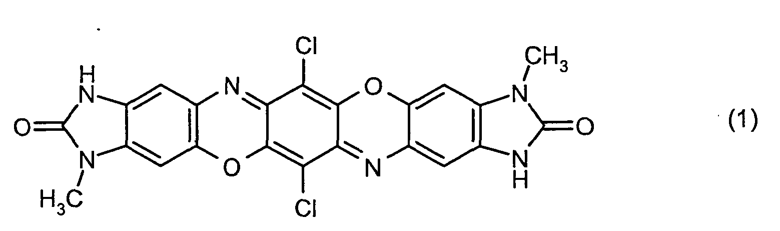 or of a tautomer thereof, which comprises causing to act on the compound of the formula (1) N-methylpyrrolidone, at a temperature of between 0 and 250°C.

2. The process as claimed in claim 1, wherein phase conversion takes place at a temperature of between 20 and 210°C.

3. The process as claimed in claim 1 or 2, wherein the compound of the formula (1) is heated to boiling in N-methylpyrrolidone.

4. A dioxazine pigment of the formula (1) 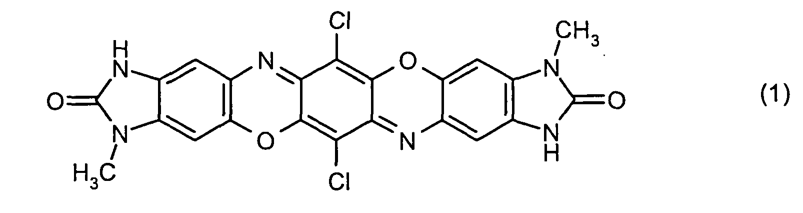 or a tautomer thereof, **characterized by** the following characteristic reflections in the X-ray powder diagram, measured with Cu-K_{α} radiation (2Θ in degrees, d in Å⁻¹):
| Phase II: | | |
|---|---|---|
| 2Θ | d | rel. intensity |
| 6.64 | 13.29 | 21 |
| 11.05 | 8.00 | 8 |
| 12.49 | 7.08 | 46 |
| 13.41 | 6.60 | 6 |
| 14.60 | 6.06 | 10 |
| 17.08 | 5.19 | 7 |
| 19.87 | 4.46 | 7 |
| 20.93 | 4.24 | 4 |
| 22.24 | 3.99 | 4 |
| 23.35 | 3.81 | 8 |
| 25.36 | 3.51 | 11 |
| 26.90 | 3.31 | 100 |
| 29.06 | 3.07 | 5 |
| 30.48 | 2.93 | 8 |
| 32.11 | 2.79 | 8 |
| 33.17 | 2.70 | 4 |

5. A mixture of a pigment of the formula (1) as claimed in claim 4, comprising at least 10% of phase II .

6. The mixture as claimed in claim 5, comprising at least 25% of phase II.

7. The mixture as claimed in claim 5, comprising at least 50 % of phase II.

8. The mixture as claimed in claim 5, comprising at least 75% of phase II.

9. The mixture as claimed in claim 5, comprising at least 90% of phase II.

10. The use of a pigment of the formula (1) as claimed in at least one of claims 4 to 9 for pigmenting coating materials, plastics, printing inks, aqueous or solvent-based pigment preparations, cosmetics, electrophotographic toners and developers, powder coating materials, inks, preferably inkjet inks, color filters, for coloring seeds, and for the pulp dyeing of paper.

## Revendications

1. Procédé de changement de phase d'un composé répondant à la formule (1) dans un pigment dioxazine selon la revendication 4 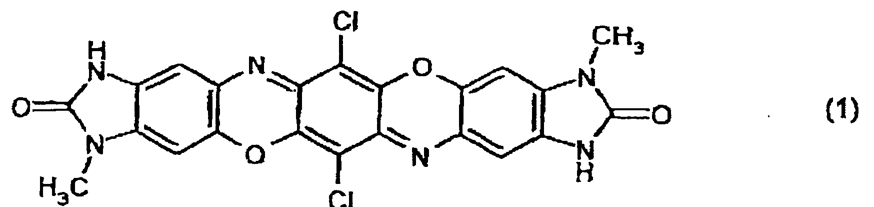 ou dans un tautomère de celui-ci,
**caractérisé en ce qu'**on fait agir sur le composé répondant à la formule (1) de la N-méthylpyrrolidone à une température de 0 à 250°C.

2. Procédé selon la revendication 1,
**caractérisé en ce que** le changement de phase se produit à une température de 20 à 210°C.

3. Procédé selon la revendication 1 ou 2,
**caractérisé en ce que** le composé répondant à la formule (1) est porté à ébullition dans la N-méthylpyrrolidone.

4. Pigment dioxazine répondant à la formule (1) 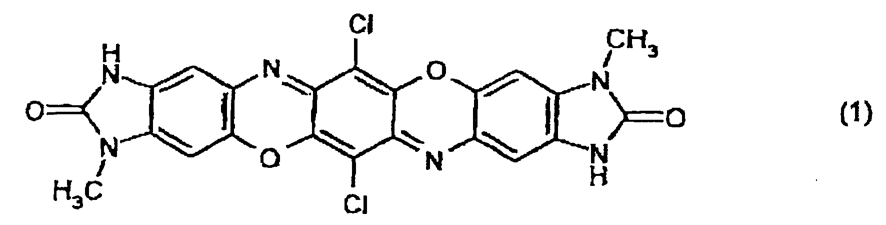 ou tautomère de celui-ci,
**caractérisé par** les réflexions caractéristiques ci-après dans le diagramme de diffraction de la poudre aux rayons X, mesurées par rayonnement Cu-K_{α} (valeurs 2Θ en degrés, valeurs d en Â⁻¹) :
| Phase II : | | |
|---|---|---|
| 2Θ | d | Intensité relative |
| 6.64 | 13.29 | 21 |
| 11.05 | 8.00 | 8 |
| 12.49 | 7.08 | 46 |
| 13.41 | 6.60 | 6 |
| 14.60 | 6.06 | 10 |
| 17.08 | 5.19 | 7 |
| 19.87 | 4.46 | 7 |
| 20.93 | 4.24 | 4 |
| 22.24 | 3.99 | 4 |
| 23.35 | 3.81 | 8 |
| 25.36 | 3.51 | 11 |
| 26.90 | 3.31 | 100 |
| 29.06 | 3.07 | 5 |
| 30.48 | 2.93 | 8 |
| 32.11 | 2.79 | 8 |
| 33.17 | 2.70 | 4 |

5. Mélange d'un pigment répondant à la formule (1) selon la revendication 4 qui contient au moins 10 % de la phase II.

6. Mélange selon la revendication 5, qui contient au moins 25 % de la phase II.

7. Mélange selon la revendication 5, qui contient au moins 50 % de la phase II.

8. Mélange selon la revendication 5, qui contient au moins 75 % de la phase II.

9. Mélange selon la revendication 5, qui contient au moins 90 % de la phase II.

10. Utilisation d'un pigment répondant à la formule (1) selon au moins une des revendications 4 à 9 pour la pigmentation de peintures, de matières plastiques, d'encres d'imprimerie, de préparations de pigments aqueuses ou contenant un solvant, de cosmétiques, de toners et de révélateurs électrophotographiques, de peintures en poudre, d'encres, de préférence des encres pour jet d'encre, d'écrans colorés, pour la coloration de semences et pour la coloration de pâte à papier.
